# EUROPÄISCHE PATENTSCHRIFT

(11) **EP 3 196 715 B1**
(45) Veröffentlichungstag und Bekanntmachung des Hinweises auf die Patenterteilung: **01.08.2018**
(21) Anmeldenummer: 16203634.7
(22) Anmeldetag: 13.12.2016
(51) Int. Cl.: G05B 19/042, G01R 33/54, A61B 6/00

(54) **STEUERUNG EINER MAGNETRESONANZANLAGE MITTELS EINER EXTERNEN STEUERVORRICHTUNG**
CONTROL OF A MAGNETIC RESONANCE SYSTEM USING AN EXTERNAL CONTROL DEVICE
COMMANDE D'UNE INSTALLATION À RÉSONANCE MAGNÉTIQUE À L'AIDE D'UN DISPOSITIF DE COMMANDE EXTERNE

(30) Priorität: 19.01.2016 DE 102016200631
(43) Veröffentlichungstag der Anmeldung: 26.07.2017
(73) Patentinhaber: Siemens Healthcare GmbH, 91052 Erlangen (DE)
(72) Erfinder: Schneider, Rainer, 91054 Erlangen (DE); Franger, Dirk, 91052 Erlangen (DE)

(56) Entgegenhaltungen:
- DE-A1- 10 065 558
- DE-A1-102011 083 957
- DE-A1-102013 105 043
- DE-A1-102014 216 669
- US-A1- 2011 013 220

## Beschreibung

Die vorliegende Erfindung betrifft die Steuerung einer Magnetresonanzanlage mit Hilfe einer externen Steuervorrichtung.

Die DE 10 2013 105 043 A1 offenbart ein modulares Steuersystem mit Berührungsbildschirmvorrichtungen, um ein medizinisches System zu steuern. Dabei wird ein Benutzer durch das System authentifiziert, um spezielle Anwendungen zu nutzen. Eine Benutzeroberfläche zeigt Patientendaten an.

Die DE 10 2011 083 957 A1 beschreibt ein elektronisches Steuerungssystem mit einem externen Ein- und Ausgabegerät und einem medizinischen Gerät, das über das externe Ein- und Ausgabegerät gesteuert wird. Sobald eine Applikation eine Eingabe zur Steuerung des medizinischen Geräts erfordert, wird diese über das Ein- und Ausgabegerät erfragt.

Eine Magnetresonanzanlage ermöglicht es, ständig Bilder eines bestimmten Volumenabschnitts eines Patienten zu erzeugen. Dadurch kann der Operateur oder Therapeut während einer Operation oder Behandlung den Patienten ständig anhand dieser Bilder überwachen. Um allerdings die Magnetresonanzanlage zu steuern, um beispielsweise die Art und Weise, wie die Bilder erfasst werden, oder den zu erfassenden Volumenabschnitt zu verändern, müssen nach dem Stand der Technik umständlich entsprechende Anweisungen über Eingabemittel der Magnetresonanzanlage eingegeben werden, welche sich nicht in der Nähe des Patienten befinden.

Daher stellt sich die vorliegende Erfindung die Aufgabe, die Steuerung einer Magnetresonanzanlage dahingehend zu verbessern, dass diese Steuerung an einer beliebigen Stelle (z.B. auch in der Nähe des Patienten) erfolgen kann.

Erfindungsgemäß wird diese Aufgabe durch ein Verfahren zum Betreiben einer Magnetresonanzanlage durch eine externe Steuervorrichtung nach Anspruch 1, durch eine Magnetresonanzanlage nach Anspruch 15, durch ein Computerprogrammprodukt nach Anspruch 18, durch einen elektronisch lesbaren Datenträger nach Anspruch 19 und durch ein System nach Anspruch 17 gelöst. Die abhängigen Ansprüche definieren bevorzugte und vorteilhafte Ausführungsformen der vorliegenden Erfindung.

Im Rahmen der vorliegenden Erfindung wird ein Verfahren zum Betreiben einer Magnetresonanzanlage durch eine externe Steuervorrichtung bereitgestellt. Dabei umfasst die Magnetresonanzanlage eine Schnittstelle, um über diese Schnittstelle eine Kommunikation mit der externen Steuervorrichtung zu betreiben. Die Magnetresonanzanlage führt folgende Schritte durch:
- Erstellen einer Kommunikationsverbindung zwischen der externen Steuervorrichtung und der Magnetresonanzanlage mittels der Schnittstelle.
- Erfassen einer Anweisung von der externen Steuervorrichtung über die Schnittstelle bzw. die Kommunikationsverbindung.
- Ausführen der Anweisung auf der Magnetresonanzanlage.

Unter einer externen Steuervorrichtung wird dabei insbesondere eine Steuervorrichtung (z.B. ein mobiles Gerät, wie ein Tablet, ein PC, ein Smartphone oder irgendein Gerät, welches zur Kommunikation über ein standardisiertes Internetprotokoll in der Lage ist) verstanden, welches räumlich getrennt von der Magnetresonanzanlage ist. Bei der Schnittstelle kann es sich um eine drahtlose oder drahtgestützte Schnittstelle handeln. Die Schnittstelle ist insbesondere standardisiert und verwendet beispielsweise ein REST ("Representational State Transfer")-basiertes HTTP-Protokoll, was der externen Steuervorrichtung ermöglicht, eine beliebige Technik einzusetzen, welche TCP/IP und HTTP unterstützt. D.h. als Kommunikationsverbindung werden LANs, WANs und alle Arten von Internet-Verbindungen unterstützt.

Bei der Anweisung, mit welcher die externe Steuervorrichtung die Magnetresonanzanlage steuert, kann es sich um eine so genannte schreibende oder ändernde Anweisung handeln, mit welcher beispielsweise eine Sequenz der Magnetresonanzanlage gesteuert oder verändert wird. Es ist aber auch möglich, dass es sich bei der Anweisung (nur) um einen so genannten Lesezugriff auf Daten oder Informationen der Magnetresonanzanlage handelt, um beispielsweise bestimmte Bilddaten auf der externen Steuervorrichtung anzeigen zu können.

Das erfindungsgemäße Verfahren ermöglicht den Einsatz einer externen Steuervorrichtung an einer beliebigen Stelle, so dass von dieser beliebigen Stelle (beispielsweise in unmittelbarer Nähe des Patienten) die Magnetresonanzanlage über diese externe Steuervorrichtung gesteuert werden kann.

Dabei werden im Rahmen der Erstellung der Kommunikationsverbindung Autorisierungsdaten der externen Steuervorrichtung von der Magnetresonanzanlage erfasst. Dabei umfassen die Autorisierungsdaten selbst eine Lizenz, die Informationen darüber enthält, welche Dienste oder Anweisungen von der Magnetresonanzanlage über die externe Steuervorrichtung ausgeführt werden können. Die jeweils erfasste Anweisung wird dann von der Magnetresonanzanlage abhängig von einer Berechtigung ausgeführt, welche durch diese Lizenz definiert wird. Dabei handelt es sich bei der Lizenz insbesondere um eine autonome ("self-contained") Lizenz, welche die jeweilige Berechtigung vollständig definiert, ohne dass die Magnetresonanzanlage zur Ermittlung der Berechtigung weitere Informationsquellen (außerhalb der Lizenz) benötigt. Die Lizenz wird insbesondere individuell für jede externe Steuervorrichtung bzw. jeden Benutzer einer externen Steuervorrichtung erzeugt.

Durch diese erfindungsgemäße Autorisierung kann sehr genau vorgegeben werden, welcher Dienst oder welche Anweisung (bzw. Operation oder Methode) der Magnetresonanzanlage von der jeweiligen externen Steuervorrichtung ausgeführt werden darf.

Dabei können mehrere Berechtigungsarten existieren, wobei die durch die Lizenz definierte Berechtigung eine oder mehrere dieser Berechtigungsarten umfasst. Jede Anweisung der Magnetresonanzanlage und/oder jeder Dienst der Magnetresonanzanlage ist dabei einer dieser Berechtigungsarten zugeordnet. Die erfasste Anweisung wird nur dann von der Magnetresonanzanlage ausgeführt,
wenn die Berechtigung diejenige Berechtigungsart umfasst, welche der Anweisung zugeordnet ist, und/oder wenn die Berechtigung diejenige Berechtigungsart umfasst, welche dem Dienst zugeordnet ist, welcher diese Anweisung bereitstellt.

Durch die vorab beschriebene Ausführungsform ermöglicht das erfindungsgemäße Verfahren eine sehr genaue Abstufung von Berechtigungen für Operationen und Methoden (Anweisungen) der Magnetresonanzanlage seitens der jeweiligen externen Steuervorrichtung. Dadurch kann auch rechtlichen und kommerziellen Bedingungen Rechnung getragen werden.

Durch die vorab beschriebene Autorisierung können sich nur autorisierte Benutzer über die externe Steuervorrichtung mit der Magnetresonanzanlage verbinden, wobei eine spezifische (d.h. Benutzer-spezifische) Lizenz, welche einer bestimmten Berechtigung zugeordnet ist, verwendet wird.

Vorteilhafterweise kann von mehreren externen Steuervorrichtungen (jeweils) eine Kommunikationsverbindung zu derselben Magnetresonanzanlage aufgebaut werden. Daher können gleichzeitig mehrere Kommunikationsverbindungen zwischen derselben Magnetresonanzanlage und verschiedenen externen Steuervorrichtungen vorhanden sein.

Diese erfindungsgemäße Ausführungsform ermöglicht, dass mehrere externe Steuervorrichtungen gleichzeitig auf dieselbe Magnetresonanzanlage zugreifen. Es können auch mehrere Prozesse oder Tasks derselben externen Steuervorrichtung gleichzeitig auf dieselbe Magnetresonanzanlage zugreifen. Beispielsweise können mit einer externen Steuervorrichtung Bilddaten erfasst werden, während gleichzeitig mit einer anderen externen Steuervorrichtung Parameter einer Sequenz derselben Magnetresonanzanlage geändert werden.

Über die Schnittstelle kann ein Informationsdienst bereitgestellt werden, welcher der externen Steuervorrichtung eine oder mehrere der folgenden Operationen bzw. Anweisungen bereitstellt:
- Erfassen einer Liste von Sequenzen, welche von der Magnetresonanzanlage ausgeführt werden können. Über diese Anweisung kann die externe Steuervorrichtung eine Liste von auf der Magnetresonanzanlage verfügbaren Sequenzen erfassen, wobei die externe Steuervorrichtung jede dieser Sequenzen öffnen und starten kann.
- Erfassen eines aktuellen Zustands einer bestimmten oder ausgewählten Sequenz. Dabei existieren für eine Sequenz folgende Zustände:
   ∘ laufend:
      Die Sequenz läuft gerade auf der Magnetresonanzanlage.
   ∘ offen:
      Die Sequenz ist geöffnet, so dass beispielsweise Parameter der Sequenz geändert werden können.
   ∘ angehalten:
      Die Sequenz wurde angehalten und kann fortgesetzt werden.
- Erfassen der verbleibenden Messzeit einer aktuell auf der Magnetresonanzanlage laufenden Sequenz. Diese Information ist insbesondere für nicht-interaktive Sequenzen von Interesse, welche keine Aktion von einem Benutzer (wie z.B. eine Tischverschiebung oder eine Atempause) erfordern, da die verbleibende Messzeit dann genau diejenige Zeitspanne angibt, die die aktuell laufende Sequenz noch bis zu ihrem Ende benötigt.

Gemäß einer weiteren erfindungsgemäßen Ausführungsform wird von der Magnetresonanzanlage über die Schnittstelle ein Steuerungsdienst bereitgestellt, welcher eine oder mehrere der folgenden Operationen oder Methoden (Anweisungen) der externen Steuervorrichtung bereitstellt:
- Öffnen einer bestimmten Sequenz. Das Öffnen der Sequenz ist eine Voraussetzung zur Änderung der Parameter dieser Sequenz.
- Schließen einer bestimmten Sequenz. Das Schließen einer geöffneten Sequenz umfasst insbesondere das Beibehalten von vorher durchgeführten Parameteränderungen oder das Verwerfen von vorher durchgeführten Parameteränderungen bezüglich der Sequenz.
- Starten der aktuell geöffneten Sequenz. Durch diese Anweisung ändert sich der Zustand der Sequenz von offen auf laufend, und es werden MR-Daten abhängig von der Sequenz mittels der Magnetresonanzanlage erfasst.
- Abbrechen der aktuell laufenden Sequenz. Durch diese Anweisung wird die Sequenz gestoppt und das Protokoll wird automatisch geschlossen. Die Sequenz kann nicht fortgesetzt, sondern nur neu gestartet werden.

Um eine der vorab beschriebenen Anweisungen des Steuerungsdienstes für eine bestimmte Sequenz auszuführen, wird die bestimmte Sequenz insbesondere vorher aus der Liste der verfügbaren Sequenzen ausgewählt, welche von dem Informationsdienst bereitgestellt wird.

Darüber hinaus kann von der Magnetresonanzanlage über die Schnittstelle ein Änderungsdienst bereitgestellt werden, welcher der externen Steuervorrichtung eine oder mehrere der folgenden Anweisungen bereitstellt:
- Ändern eines oder mehrerer Parameter einer bestimmten Sequenz. Dabei können auch die Parameter einer aktuell laufenden Sequenz geändert werden.
- Anhalten der aktuell laufenden Sequenz. Das Anhalten oder Pausieren einer Sequenz bedeutet, dass die Sequenz aktuell keine MR-Daten erfasst, so dass die Magnetresonanzanlage lautlos ist. Im Gegensatz zu einer abgebrochenen Sequenz kann eine angehaltene Sequenz fortgesetzt werden.
- Fortsetzen der aktuell angehaltenen Sequenz. Die vorher angehalten Sequenz wird fortgesetzt, so dass die Sequenz wieder MR-Daten erfasst.

Der erfindungsgemäße Änderungsdienst ermöglicht der externen Steuervorrichtung durch eine entsprechende Änderung der Parameter einer Sequenz die Messeigenschaften der Sequenz zu ändern, um so das Ergebnis (insbesondere die anhand der mittels der Sequenz erfassten MR-Daten erzeugten MR-Bilder) zu beeinflussen und/oder zu ändern. Dabei können auch die Parameter der aktuell laufenden Sequenz von der externen Steuervorrichtung geändert werden. Dabei kann die Menge der von der externen Steuervorrichtung zu ändernden Parameter eingeschränkt sein, wie es im Folgenden ausgeführt wird.

Darüber hinaus kann von der Magnetresonanzanlage über die Schnittstelle ein Patientendatendienst bereitgestellt werden, über welchen die externe Steuervorrichtung eine oder mehrere der folgenden Informationen erfassen kann:
- Den Namen des Patienten.
- Das Alter des Patienten.
- Die Körperlänge des Patienten.
- Das Gewicht des Patienten.

Anhand dieser Patientendaten kann beispielsweise die Sequenz, mit welcher die MR-Bilder des Patienten erzeugt werden, an den Patienten angepasst werden. Der Zugriff auf diese Patientendaten über die externe Steuervorrichtung ist vorteilhafterweise nur Benutzern mit einer entsprechenden Berechtigung vorbehalten.

Gemäß einer weiteren erfindungsgemäßen Ausführungsform kann von der Magnetresonanzanlage über die Schnittstelle ein Interaktionsdienst bereitgestellt werden, über welchen eine Meldung oder ein Hinweis, welcher von der Magnetresonanzanlage ausgegeben wird, an die externe Steuervorrichtung weitergeleitet wird.

Über diesen erfindungsgemäßen Interaktionsdienst können Hinweise und/oder Meldungen von der Magnetresonanzanlage an die externe Steuervorrichtung weitergeleitet werden, welche über eine Aktion der Magnetresonanzanlage informieren, die u.U. einen Dialogeingriff eines Benutzers erfordert. Dies kann beispielsweise einen Hinweis oder eine Meldung bzw. eine Information über eine anstehende Tischbewegung, über eine automatische Anpassung des aktuell offenen Protokolls oder eine Warnung über eine Nerven-Stimulation (z.B. durch die schnell wechselnden Gradienten-Magnetfelder) oder die Verabreichung eines Kontrastmittels an den Patienten umfassen.

Wenn der Hinweis oder die Meldung eine Bestätigung erfordert, damit die aktuell angehaltene Sequenz der Magnetresonanzanlage fortgesetzt wird, kann diese Bestätigung von der externen Steuervorrichtung an die Magnetresonanzanlage weitergeleitet werden.

Beispielsweise kann die Steuervorrichtung periodisch abfragen, ob eine Bestätigung erforderlich ist. Wenn dies der Fall ist, kann die externe Steuervorrichtung die von der Magnetresonanzanlage über den Interaktionsdienst erfasste Meldung auf ihrer Anzeige darstellen, um dann eine entsprechende Benutzereingabe (z.B. Bestätigung oder Abbruch) zu erfassen und an die Magnetresonanzanlage weiterzuleiten.

Der Interaktionsdienst ist insbesondere generisch aufgebaut. Die externe Steuervorrichtung hat daher über die Semantik der über den Interaktionsdienst erfassten Meldung keine Kenntnis, sondern zeigt auf ihrer Anzeige nur die übermittelte Meldung und die Möglichkeiten der Antwort (Bestätigung und ggfls. Abbruch) an. Die vom Benutzer der externen Steuervorrichtung eingegebene Antwort, wird dann an die Magnetresonanzanlage übermittelt. Falls die Meldung vor dem Start der entsprechenden Sequenz übermittelt wird, führt ein Abbruch dazu, dass die Sequenz nicht gestartet wird. Eine Antwort (auch eine Abbruch-Antwort) auf die Meldung muss nicht zwangsläufig eine Auswirkung auf die Durchführung oder Weiterführung der Sequenz nach sich ziehen. Beispielsweise kann eine Abbruch-Antwort auch nur dazu führen, dass eine Verschiebung des Tisches nicht durchgeführt wird.

Gemäß einer weiteren erfindungsgemäßen Ausführungsform wird von der Magnetresonanzanlage über die Schnittstelle ein Parameterdienst bereitgestellt, über welchen der externen Steuervorrichtung eine Schnittstelle bereitgestellt wird, um über diese Schnittstelle Parameterinformationen bezüglich Parametern von Sequenzen der Magnetresonanzanlage zu erfassen.

Über die von dem Parameterdienst bereitgestellte Schnittstelle können demnach nicht nur Parameter, Teilmengen von Parametern, sondern auch Informationen über die Parameter bereitgestellt werden.

Beispielsweise können der externen Steuervorrichtung von der Magnetresonanzanlage über diese Schnittstelle abhängig von einer bestimmten Sequenz eine Teilmenge derjenigen Parameter bereitgestellt werden, welche von der externen Steuervorrichtung bezüglich dieser bestimmten Sequenz geändert werden dürfen.

Indem der externen Steuervorrichtung nur diejenigen Parameter in Form der Teilmenge bereitgestellt werden, welche geändert werden dürfen, ist vorteilhafterweise sichergestellt, dass die externe Steuervorrichtung bei dieser Ausführungsform andere Parameter, welche vom durchschnittlichen Benutzer bezüglich der bestimmten Sequenz nicht geändert werden sollten, nicht ändert. Diese Teilmenge der änderbaren Parameter unterscheidet sich dabei in der Regel zwischen den verschiedenen Sequenzen, da jede Sequenz ihre individuellen Eigenschaften und Anforderungen aufweist.

Die Parameterinformation kann eine oder kann mehrere Informationen aus einer Informationsgruppe umfassen, wobei die Informationsgruppe selbst folgende Informationen umfasst:
- Eine Information über die Verfügbarkeit und Änderbarkeit des Parameters. Diese Information gibt an, ob der jeweilige Parameter für die entsprechende Sequenz vorhanden ist und ob der jeweilige Parameter für die entsprechende Sequenz geändert werden darf.
- Eine Bezeichnung des Parameters. Dabei wird vorteilhafterweise eine international übliche Bezeichnung eingesetzt.
- Einen Typ und eine Einheit des Parameters.
- Gültige Werte des Parameters. Wenn der Parameterwert ein Element einer endlichen Liste (z.B. "ja", "nein") ist, wird diese endliche Liste der gültigen Parameterwerte der externen Steuervorrichtung bereitgestellt.
- Eine Information über einen einstellbaren Wertebereich des Parameters. Diese Information kann beispielsweise einen Minimalwert und einen Maximalwert umfassen, zwischen welchen ein gültiger Wert für den Parameter liegt.
- Eine detaillierte Beschreibung des Parameters. Diese Beschreibung dient zur genauen Definition des Parameters.
- Eine eindeutige Identifikation des Parameters. Über diese Identifikation kann der Parameter bei einer Anweisung, welche die externe Steuervorrichtung an die Magnetresonanzanlage sendet, eindeutig referenziert werden.

Die Schnittstelle des Parameterdiensts stellt vorteilhafterweise auch die Möglichkeit bereit, eine vorab beschriebene Teilmenge der Parameter zu erzeugen und zu ändern. Mit anderen Worten kann für eine bestimmte Sequenz eine Teilmenge erzeugt oder geändert werden, welche diejenigen Parameter der Sequenz umfasst, die von der externen Steuervorrichtung (später) bezüglich dieser Sequenz verändert werden können.

Diese Konfiguration der Teilmengen derjenigen Parameter, welche von der externen Steuervorrichtung bezüglich einer bestimmten Sequenz geändert werden können, kann beispielsweise nur dann bereitgestellt werden, wenn die externe Steuervorrichtung oder genauer der Benutzer der externen Steuervorrichtung eine entsprechende Berechtigung aufweist. Diese Änderung oder Erzeugung einer Teilmenge kann dabei auch das Erzeugen und Ändern der vorab beschriebenen Parameterinformation pro Parameter der jeweiligen Teilmenge umfassen.

Im Normalfall obliegt das Erzeugen und Ändern der Teilmengen derjenigen Parameter, welche von der externen Steuervorrichtung bezüglich einer bestimmten Sequenz geändert werden können, einem Spezialisten, so dass es auch ausreicht wenn dieses Erzeugen und Ändern nur direkt an der Magnetresonanzanlage (und nicht über eine externe Steuereinrichtung) möglich ist.

Darüber hinaus kann abhängig von den Autorisierungsdaten (d.h. abhängig von der Berechtigung) jeder Parameter irgendeiner Sequenz der Magnetresonanzanlage über die externe Steuervorrichtung geändert werden.

Bei der Schnittstelle, welche von dem erfindungsgemäßen Parameterdienst bereitgestellt wird, handelt es sich vorteilhafterweise um eine generische Schnittstelle. Dadurch kann die externe Steuervorrichtung auf einheitliche Weise auf jeden beliebigen Parameter eine Sequenz zugreifen, ohne irgendwelche inhärenten Eigenschaften des Parameters kennen zu müssen. Dadurch kann vorteilhafterweise auch die Teilmenge derjenigen Parameter, welche von der externen Steuervorrichtung bezüglich einer bestimmten Sequenz geändert werden dürfen, in beliebiger Weise erweitert werden, ohne dass dazu eine Änderung der Software der externen Steuervorrichtung notwendig ist. Mit anderen Worten kann dieser Teilmenge auch ein Parameter zugefügt werden, welcher der externen Steuervorrichtung bis dahin noch nicht bekannt ist. Auch die Anzahl der Parameter innerhalb dieser Teilmenge ist frei einstellbar, ohne dass irgendwelche Änderungen an der Software der externen Steuervorrichtung notwendig sind. Aufgrund der generischen Schnittstelle benötigt die externe Steuervorrichtung keinerlei semantisches Vorwissen über die Anzahl und die Art der Parameter sowie kein internes Vorwissen über die Bedeutung und die Bezeichnung eines Parameters. Jegliche notwendige Information zur Ausführung der vorab beschriebenen Anweisungen wird der externen Steuervorrichtung über die generische Schnittstelle bereitgestellt.

Im Rahmen der vorliegenden Erfindung wird auch eine Magnetresonanzanlage bereitgestellt. Dabei umfasst die Magnetresonanzanlage einen Grundfeldmagneten, ein Gradientenfeldsystem, mindestens eine HF-Antenne, eine Schnittstelle zur Kommunikation mit einer externen Steuervorrichtung und eine Steuereinrichtung zur Ansteuerung des Gradientenfeldsystems und der mindestens einen HF-Antenne, zum Empfang von von der oder den HF-Antennen aufgenommenen Messsignalen und zur Auswertung der Messsignale. Die Magnetresonanzanlage ist derart ausgestaltet, dass die Magnetresonanzanlage über die Schnittstelle eine Anweisung der externen Steuervorrichtung erfasst und diese Anweisung ausführt.

Die Vorteile der erfindungsgemäßen Magnetresonanzanlage entsprechen dabei im Wesentlichen den Vorteilen des erfindungsgemäßen Verfahrens, welche vorab im Detail ausgeführt worden sind, so dass hier auf eine Wiederholung verzichtet wird.

Des Weiteren beschreibt die vorliegende Erfindung ein Computerprogrammprodukt, insbesondere ein Computerprogramm oder eine Software, welche man in einen Speicher einer programmierbaren Steuerung bzw. einer Recheneinheit einer Magnetresonanzanlage laden kann. Mit diesem Computerprogrammprodukt können alle oder verschiedene vorab beschriebene Ausführungsformen des erfindungsgemäßen Verfahrens ausgeführt werden, wenn das Computerprogrammprodukt in der Steuerung oder Steuereinrichtung der Magnetresonanzanlage läuft. Dabei benötigt das Computerprogrammprodukt eventuell Programmmittel, z.B. Bibliotheken und Hilfsfunktionen, um die entsprechenden Ausführungsformen der Verfahren zu realisieren. Mit anderen Worten soll mit dem auf das Computerprogrammprodukt gerichteten Anspruch insbesondere ein Computerprogramm oder eine Software unter Schutz gestellt werden, mit welcher eine der oben beschriebenen Ausführungsformen des erfindungsgemäßen Verfahrens ausgeführt werden kann bzw. welche diese Ausführungsform ausführt. Dabei kann es sich bei der Software um einen Quellcode (z.B. C++), der noch kompiliert (übersetzt) und gebunden oder der nur interpretiert werden muss, oder um einen ausführbaren Softwarecode handeln, der zur Ausführung nur noch in die entsprechende Recheneinheit bzw. Steuereinrichtung zu laden ist.

Des Weiteren offenbart die vorliegende Erfindung einen elektronisch lesbaren Datenträger, z.B. eine DVD, ein Magnetband, eine Festplatte oder einen USB-Stick, auf welchem elektronisch lesbare Steuerinformationen, insbesondere Software (vgl. oben), gespeichert ist. Wenn diese Steuerinformationen (Software) von dem Datenträger gelesen und in eine Steuereinrichtung bzw. Recheneinheit einer Magnetresonanzanlage gespeichert werden, können alle erfindungsgemäßen Ausführungsformen des vorab beschriebenen Verfahrens durchgeführt werden.

Schließlich offenbart die vorliegende Erfindung ein System, welches eine erfindungsgemäße Magnetresonanzanlage eine externe Steuervorrichtung umfasst. Dabei umfasst die Steuervorrichtung selbst eine Steuerung, eine Schnittstelle zur Kommunikation mit einer Magnetresonanzanlage und einer Anzeige. Die Steuervorrichtung ist ausgestaltet, um über die Schnittstelle eine Kommunikationsverbindung mit der Magnetresonanzanlage zu erstellen und um eine Anweisung über die Schnittstelle an die Magnetresonanzanlage zu senden.

Die Vorteile des erfindungsgemäßen Systems entsprechen im Wesentlichen den Vorteilen des erfindungsgemäßen Verfahrens, welche vorab im Detail ausgeführt sind, so dass hier auf eine Wiederholung verzichtet wird.

Die vorliegende Erfindung weist folgende Vorteile auf:
- Die Erfindung ermöglicht ein abgesetztes Steuern einer vollständigen MR-Untersuchung über die externe Steuervorrichtung.
- Alle relevanten Informationen über die aktuell laufende oder eine offene Sequenz, die Liste der verfügbaren Sequenzen und die Patientendaten können auf der externe Steuervorrichtung dargestellt werden.
- Es ist kein direkter Dialog mit der Magnetresonanzanlage erforderlich. D. h. der Benutzer (z.B. der Operateur oder Therapeut) kann die gesamte Behandlung einschließlich der gesamten MR-Bildgebung über die externe Steuervorrichtung ausführen, ohne den Patienten allein zu lassen oder Hilfe von einer anderen Person anzufordern, welche die Magnetresonanzanlage direkt steuert.
- Es können mehrere externe Steuervorrichtungen gleichzeitig mit der Magnetresonanzanlage verbunden sein.
- Die Kommunikation zwischen der externen Steuervorrichtung und der Magnetresonanzanlage kann auf einem standardisierten Protokoll basieren, was die Integration von weiteren Diensten erleichtert.
- Ein Spezialist ist allein mit der externen Steuervorrichtung in der Lage, die für eine externe Steuervorrichtung verfügbaren Parameter für jede Sequenz abhängig von dem Typ der Sequenz sowie abhängig von dem Zusammenhang und der Anwendung, in welchem/r die Sequenz eingesetzt wird, zu konfigurieren.
- Die erfindungsgemäße Software einer externen Steuervorrichtung muss beispielsweise aufgrund von Ausführungsbestimmungen nicht geändert werden. Es reicht stattdessen aus, die Parameterinformationen auf der Seite der Magnetresonanzanlage zu ändern und über die entsprechende Schnittstelle der externen Steuervorrichtung bereitzustellen.

Die vorliegende Erfindung ermöglicht folgende Aktionen über die externe Steuervorrichtung:
- Eigenschaften der aktuell laufenden MR-Sequenz (z.B. Schichtposition, Schicht-Ausrichtung) können geändert werden.
- Die aktuell laufende MR-Sequenz kann angehalten und fortgesetzt werden.
- Eine Liste der von der Magnetresonanzanlage verfügbaren MR-Sequenzen kann geöffnet werden, wobei die geöffnete Sequenz geändert und gestartet werden kann.
- Die aktuell laufende MR-Sequenz kann abgebrochen werden.
- Der Name der aktuell geöffneten oder aktuell laufenden MR-Sequenz kann angezeigt werden.
- Die verbleibende Messzeit einer laufenden nicht-interaktiven MR-Sequenz kann angezeigt werden.
- Die Eigenschaften einer geöffneten MR-Sequenz können editiert werden, während eine andere nicht-interaktive MR-Sequenz läuft.
- Meldungen der Magnetresonanzanlage, z.B. eine Warnung vor einer Tischbewegung oder vor einer automatischen Anpassung der MR-Sequenz, können angezeigt und bestätigt werden.

Im Folgenden wird die vorliegende Erfindung anhand erfindungsgemäßer Ausführungsformen mit Bezug zu den Figuren im Detail erläutert.
In Fig. 1 ist schematisch eine erfindungsgemäße Magnetresonanzanlage in einer Kommunikationsverbindung mit einer externen Steuervorrichtung dargestellt.
In Fig. 2 ist eine erfindungsgemäße Softwarekomponente der Magnetresonanzanlage im Zusammenspiel mit externen Steuervorrichtungen dargestellt.
In Fig. 3 ist ein Menü dargestellt, um die bezüglich einer Sequenz änderbaren Parameter gemäß der vorliegenden Erfindung vorzugeben.
In Fig. 4 ist eine beispielhafte Belegung der änderbaren Parameter der Fig. 3 dargestellt.

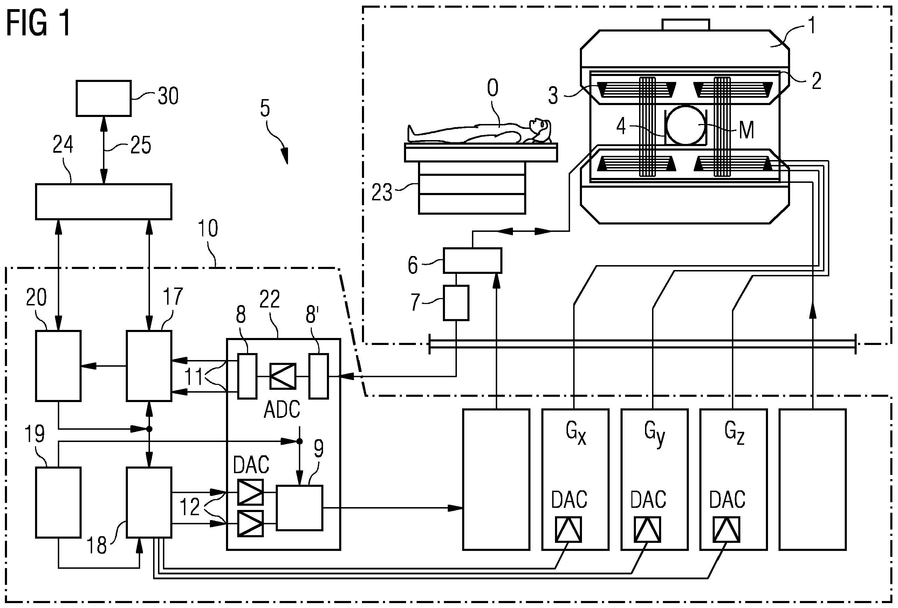

In Fig. 1 ist eine erfindungsgemäße Magnetresonanzanlage 5 (ein Magnetresonanz-Bildgebungs- bzw. Kernspintomographiegerät) in einer Kommunikationsverbindung 25 mit einer externen Steuervorrichtung 30 dargestellt. Dabei erzeugt ein Grundfeldmagnet 1 der Magnetresonanzanlage 5 ein zeitlich konstantes starkes Magnetfeld zur Polarisation bzw. Ausrichtung der Kernspins in einem Untersuchungsbereich eines Objekts O, wie z.B. eines zu untersuchenden Teils eines menschlichen Körpers, welcher auf einem Tisch 23 liegend in der Magnetresonanzanlage 5 untersucht wird. Die für die Kernspinresonanzmessung erforderliche hohe Homogenität des Grundmagnetfelds ist in einem typischerweise kugelförmigen Messvolumen M definiert, in welchem der zu untersuchende Volumenabschnitt des menschlichen Körpers angeordnet ist. Zur Unterstützung der Homogenitätsanforderungen und insbesondere zur Eliminierung zeitlich invariabler Einflüsse werden an geeigneter Stelle so genannte Shim-Bleche aus ferromagnetischem Material angebracht. Zeitlich variable Einflüsse werden durch Shim-Spulen 2 eliminiert.

In den Grundfeldmagneten 1 ist ein zylinderförmiges Gradientenfeldsystem bzw. Gradientenfeldsystem 3 eingesetzt, welches aus drei Teilwicklungen besteht. Jede Teilwicklung wird von einem Verstärker mit Strom zur Erzeugung eines linearen (auch zeitlich veränderbaren) Gradientenfeldes in die jeweilige Richtung des kartesischen Koordinatensystems versorgt. Die erste Teilwicklung des Gradientenfeldsystems 3 erzeugt dabei einen Gradienten Gₓ in x-Richtung, die zweite Teilwicklung einen Gradienten G_{y} in y-Richtung und die dritte Teilwicklung einen Gradienten G_{z} in z-Richtung. Der Verstärker umfasst einen Digital-Analog-Wandler, welcher von einer Sequenzsteuerung 18 zum zeitrichtigen Erzeugen von Gradientenpulsen angesteuert wird.

Innerhalb des Gradientenfeldsystems 3 befindet sich eine (oder mehrere) Hochfrequenzantennen 4, welche die von einem Hochfrequenzleistungsverstärker abgegebenen Hochfrequenzpulse in ein magnetisches Wechselfeld zur Anregung der Kerne und Ausrichtung der Kernspins des zu untersuchenden Objekts O bzw. des zu untersuchenden Bereiches des Objekts O umsetzen. Jede Hochfrequenzantenne 4 besteht aus einer oder mehreren HF-Sendespulen und einer oder mehreren HF-Empfangsspulen in Form einer ringförmigen vorzugsweise linearen oder matrixförmigen Anordnung von Komponentenspulen. Von den HF-Empfangsspulen der jeweiligen Hochfrequenzantenne 4 wird auch das von den präzedierenden Kernspins ausgehende Wechselfeld, d.h. in der Regel die von einer Pulssequenz aus einem oder mehreren Hochfrequenzpulsen und einem oder mehreren Gradientenpulsen hervorgerufenen Kernspinechosignale, in eine Spannung (Messsignal) umgesetzt, welche über einen Verstärker 7 einem Hochfrequenz-Empfangskanal 8 eines Hochfrequenzsystems 22 zugeführt wird. Das Hochfrequenzsystem 22, welches Teil einer Steuereinrichtung 10 der Magnetresonanzanlage 5 ist, umfasst weiterhin einen Sendekanal 9, in welchem die Hochfrequenzpulse für die Anregung der magnetischen Kernresonanz erzeugt werden. Dabei werden die jeweiligen Hochfrequenzpulse aufgrund einer vom Anlagerechner 20 vorgegebenen Pulssequenz in der Sequenzsteuerung 18 digital als Folge komplexer Zahlen dargestellt. Diese Zahlenfolge wird als Real- und als Imaginärteil über jeweils einen Eingang 12 einem Digital-Analog-Wandler im Hochfrequenzsystem 22 und von diesem einem Sendekanal 9 zugeführt. Im Sendekanal 9 werden die Pulssequenzen einem Hochfrequenz-Trägersignal aufmoduliert, dessen Basisfrequenz der Resonanzfrequenz der Kernspins im Messvolumen entspricht.

Die Umschaltung von Sende- auf Empfangsbetrieb erfolgt über eine Sende-/Empfangsweiche 6. Die HF-Sendespulen der Hochfrequenzantenne(n) 4 strahlt/en die Hochfrequenzpulse zur Anregung der Kernspins in das Messvolumen M ein und resultierende Echosignale werden über die HF-Empfangsspule(n) abgetastet. Die entsprechend gewonnenen Kernresonanzsignale werden im Empfangskanal 8' (erster Demodulator) des Hochfrequenzsystems 22 phasenempfindlich auf eine Zwischenfrequenz demoduliert, im Analog-Digital-Wandler (ADC) digitalisiert und über den Ausgang 11 ausgegeben. Dieses Signal wird noch auf die Frequenz 0 demoduliert. Die Demodulation auf die Frequenz 0 und die Trennung in Real- und Imaginärteil findet nach der Digitalisierung in der digitalen Domäne in einem zweiten Demodulator 8 statt. Durch einen Bildrechner 17 wird aus den dergestalt über einen Ausgang 11 gewonnenen Messdaten ein MR-Bild rekonstruiert. Die Verwaltung der Messdaten, der Bilddaten und der Steuerprogramme erfolgt über den Anlagenrechner 20. Aufgrund einer Vorgabe mit Steuerprogrammen kontrolliert die Sequenzsteuerung 18 die Erzeugung der jeweils gewünschten Pulssequenzen und das entsprechende Abtasten des k-Raumes. Insbesondere steuert die Sequenzsteuerung 18 dabei das zeitrichtige Schalten der Gradienten, das Aussenden der Hochfrequenzpulse mit definierter Phasenamplitude sowie den Empfang der Kernresonanzsignale. Die Zeitbasis für das Hochfrequenzsystem 22 und die Sequenzsteuerung 18 wird von einem Synthesizer 19 zur Verfügung gestellt. Die Auswahl entsprechender Sequenzen oder Steuerprogramme zur Erzeugung eines MR-Bildes kann beispielsweise über die externe Steuervorrichtung 30 vorgenommen werden, welche über eine genormte Kommunikationsschnittstelle 24 in Verbindung mit der erfindungsgemäßen Magnetresonanzanlage 5 steht.

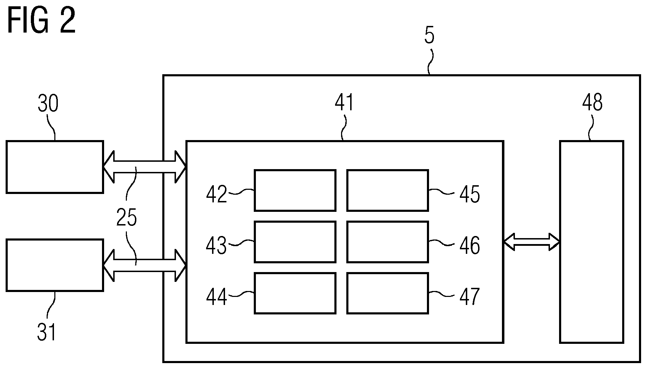

In Fig. 2 ist im Wesentlichen eine erfindungsgemäße Softwarekomponente 41 der Magnetresonanzanlage 5 im Zusammenspiel mit zwei externen Steuervorrichtungen 30, 31 dargestellt. Die Softwarekomponente 41, welche auf Rechnermitteln der Magnetresonanzanlage 5 läuft, umfasst einen Autorisierungsdienst 42, einen Patientendatendienst 43, einen Änderungsdienst 44, einen Informationsdienst 45, einen Steuerungsdienst 46 und einen Interaktionsdienst 47. Die Softwarekomponente 41 ist dabei mit einer Programm-Warteschlange 48 verbunden. Die beiden externen Steuervorrichtungen 30, 31 sind jeweils über eine Kommunikationsverbindung 25 kommunikationstechnisch mit der Magnetresonanzanlage 5 verbunden.

Über diese Kommunikationsverbindung 25 kann die jeweilige externe Steuervorrichtung 30, 31 eine Anweisung an die Magnetresonanzanlage 5 absetzen, welche von einem der Dienste 42-47 bereitgestellt wird. Abhängig von der Berechtigung des Benutzers der externen Steuervorrichtung 30, 31 wird diese Anweisung dann von dem jeweiligen Dienst 42-47 mittels der Magnetresonanzanlage 5 ausgeführt.

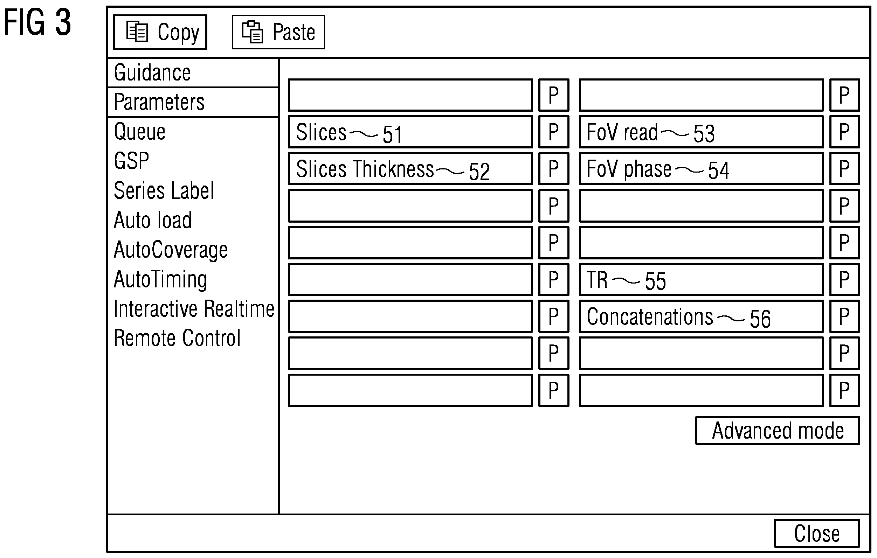

In Fig. 3 ist ein Menü dargestellt, um diejenigen Parameter 51-56, welche für eine bestimmte Sequenz von der externen Steuervorrichtung geändert werden dürfen, zu definieren. Dabei beschreiben "Slices" 51 die Anzahl der zu erfassenden Schichten, "Slice thickness" 52 die jeweilige Schichtdicke, "FoV read" 53 die Länge der Schicht in Ausleserichtung, "FoV phase" 54 die Länge der Schicht in Phasenkodierrichtung, "TR" 55 die Repetitionszeit und "Concatenation" 56 die Anzahl der Wiederholungen. Auf dieses Menü kann im Normalfall nicht über die externe Steuervorrichtung zugegriffen werden.

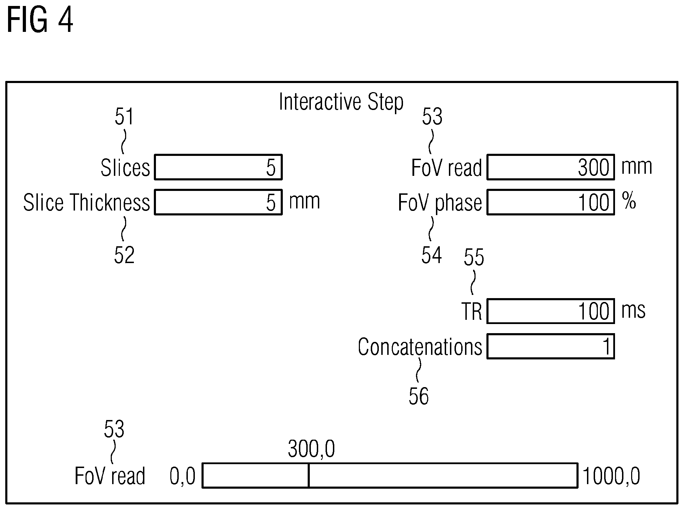

In Fig. 4 sind beispielhaft jeweils Parameterwerte für die in Fig. 3 dargestellten Parameter 51-56 abgebildet. Diese in Fig. 4 dargestellte Eingabemaske wird insbesondere zur Eingabe der Parameterwerte über die externe Steuervorrichtung 30, 31 eingesetzt.

Im Folgenden wird ein typischer erfindungsgemäßer Arbeitsablauf dargestellt.
1. Eine externe Steuervorrichtung registriert sich bei der Magnetresonanzanlage mit einer gültigen Lizenz, welche von dem Autorisierungsdienst bewertet wird. Die Berechtigung der externen Steuervorrichtung oder genauer des Benutzers der externen Steuervorrichtung wird anhand der Lizenz von dem Autorisierungsdienst ermittelt. Die folgenden Schritte werden nur ausgeführt, wenn die Berechtigung ausreichend hoch für die entsprechende Aktion bzw. Operation ist.
2. Die externe Steuervorrichtung erfasst die Liste der verfügbaren Sequenzen der Magnetresonanzanlage mittels des Informationsdienstes.
3. Mit Hilfe des Steuerungsdienstes öffnet die externe Steuervorrichtung eine vorher aus der oben genannten Liste ausgewählte interaktive Sequenz. Anschließend modifiziert die externe Steuervorrichtung verschiedene Parameter dieser Sequenz über den Änderungsdienst und startet die Sequenz über den Steuerungsdienst. Bei einer laufenden interaktiven Sequenz können die Parameter geändert werden, wodurch eine Änderung in Echtzeit ermöglicht wird. Eine laufende Sequenz kann über den Änderungsdienst angehalten (d.h. die Sequenz pausiert und die Magnetresonanzanlage erfasst keine MR-Daten) und später fortgesetzt werden.
4. Nach einer bestimmten Zeit bricht die externe Steuervorrichtung mittels des Steuerungsdienstes die laufende Sequenz ab, öffnet eine nicht-interaktive Sequenz, modifiziert einige Parameter dieser Sequenz und startet diese Sequenz. Das Protokoll einer nicht-interaktiven Sequenz wird bei dieser Ausführungsform sofort geschlossen, wenn die Sequenz gestartet wird.
5. Während die nicht-interaktive Sequenz läuft, öffnet die externe Steuervorrichtung eine andere Sequenz und modifiziert deren Parameter. Um die geöffnete Sequenz zu starten, muss die aktuell laufende Sequenz entweder über den Steuerungsdienst abgebrochen werden oder es muss gewartet werden, bis die laufende Sequenz beendet ist.
6. Die externe Steuervorrichtung meldet sich von der Magnetresonanzanlage ab.

## Patentansprüche

1. Verfahren zum Betreiben einer Magnetresonanzanlage (5) durch eine externe Steuervorrichtung (30; 31),
wobei die Magnetresonanzanlage (5) eine Schnittstelle (24) zur Kommunikation mit der externen Steuervorrichtung (30; 31) aufweist, wobei die Magnetresonanzanlage (5) folgende Schritte durchführt:
Erstellen einer Kommunikationsverbindung (25) zwischen der externen Steuervorrichtung (30; 31) und der Magnetresonanzanlage (5) über die Schnittstelle (24),
Erfassen einer Anweisung von der externen Steuervorrichtung (30; 31) über die Schnittstelle (24), und
Ausführen der Anweisung auf der Magnetresonanzanlage (5).
**dadurch gekennzeichnet,**
**dass** das Erstellen der Kommunikationsverbindung (25) ein Erfassen von Autorisierungsdaten der externen Steuervorrichtung (30; 31) umfasst, wobei die Autorisierungsdaten eine Lizenz aufweisen, die angibt, welche Dienste (42-47) oder Anweisungen in der Magnetresonanzanlage (5) von der externen Steuervorrichtung (30; 31) ausgeführt werden können, und
**dass** die erfasste Anweisung von der Magnetresonanzanlage (5) abhängig von einer Berechtigung, welche durch die Lizenz definiert wird, ausgeführt wird.

2. Verfahren nach Anspruch 1,
**dadurch gekennzeichnet,**
**dass** mehrere Berechtigungsarten existieren,
**dass** die Berechtigung mindestens eine der Berechtigungsarten umfasst,
**dass** jede Anweisung der Magnetresonanzanlage (5) und/oder jeder Dienst (42-47) der Magnetresonanzanlage (5) einer der Berechtigungsarten zugeordnet ist, und
**dass** die erfasste Anweisung von der Magnetresonanzanlage (5) nur ausgeführt wird, wenn die Berechtigung die der Anweisung zugeordnete Berechtigungsart und/oder die einem Dienst (42-47), welcher der Anweisung zugeordnet ist, zugeordnete Berechtigungsart aufweist.

3. Verfahren nach einem der vorhergehenden Ansprüche,
**dadurch gekennzeichnet,**
**dass** die Magnetresonanzanlage (5) mehrere Kommunikationsverbindungen (25) zu mehreren externen Steuervorrichtungen (30, 31) erstellt, so dass gleichzeitig mehrere Kommunikationsverbindungen (25) zwischen jeweils einer externen Steuervorrichtung (30; 31) und der Magnetresonanzanlage (5) vorhanden sind.

4. Verfahren nach einem der vorhergehenden Ansprüche,
**dadurch gekennzeichnet,**
**dass** von der Magnetresonanzanlage (5) über die Schnittstelle (24) ein Informationsdienst (45) bereitgestellt wird, mit welchem mindestens eine der folgenden Anweisungen ausführbar ist:
Erfassen einer Liste von Sequenzen, welche für die Magnetresonanzanlage (5) verfügbar sind,
Erfassen eines aktuellen Zustands einer bestimmten Sequenz, und
Erfassen einer verbleibenden Messzeit einer aktuell auf der Magnetresonanzanlage (5) laufenden Sequenz.

5. Verfahren nach einem der vorhergehenden Ansprüche,
**dadurch gekennzeichnet,**
**dass** von der Magnetresonanzanlage (5) über die Schnittstelle (24) ein Steuerungsdienst (46) bereitgestellt wird, mit welchem mindestens eine der folgenden Anweisungen ausführbar ist:
Öffnen einer bestimmten Sequenz,
Schließen einer bestimmten Sequenz,
Starten einer geöffneten Sequenz, und
Abbrechen einer gestarteten Sequenz.

6. Verfahren nach einem der vorhergehenden Ansprüche,
**dadurch gekennzeichnet,**
**dass** von der Magnetresonanzanlage (5) über die Schnittstelle (24) ein Änderungsdienst (44) bereitgestellt wird, mit welchem mindestens eine der folgenden Anweisungen ausführbar ist:
Ändern mindestens eines Parameters einer bestimmten Sequenz, und
Anhalten einer laufenden Sequenz, und
Fortsetzen einer angehaltenen Sequenz.

7. Verfahren nach einem der vorhergehenden Ansprüche,
**dadurch gekennzeichnet,**
**dass** von der Magnetresonanzanlage (5) über die Schnittstelle (24) ein Patientendatendienst (43) bereitgestellt wird, über welchen mindestens eine von folgenden Informationen erfassbar ist:
der Name des Patienten,
das Alter des Patienten,
die körperliche Länge des Patienten, und
das Gewicht des Patienten.

8. Verfahren nach einem der vorhergehenden Ansprüche,
**dadurch gekennzeichnet,**
**dass** von der Magnetresonanzanlage (5) über die Schnittstelle (24) ein Interaktionsdienst (47) bereitgestellt wird, welcher eine Meldung, welche von der Magnetresonanzanlage (5) ausgegeben wird, an die externe Steuervorrichtung (30; 31) weiterleitet.

9. Verfahren nach Anspruch 8,
**dadurch gekennzeichnet,**
**dass** die Meldung eine Bestätigung erfordert, damit eine aktuelle Sequenz der Magnetresonanzanlage (5) weiter ausgeführt wird, und
**dass** die Bestätigung von der externen Steuervorrichtung (5) erfasst wird.

10. Verfahren nach einem der vorhergehenden Ansprüche,
**dadurch gekennzeichnet,**
**dass** von der Magnetresonanzanlage über die Schnittstelle ein Parameterdienst bereitgestellt wird, welcher der externen Steuervorrichtung (30; 31) eine Schnittstelle zur Verfügung stellt, um über diese Schnittstelle Parameterinformationen bezüglich Parametern (51-56) von Sequenzen der Magnetresonanzanlage (5) zu erfassen.

11. Verfahren nach Anspruch 10,
**dadurch gekennzeichnet,**
**dass** von der Magnetresonanzanlage (5) der externen Steuervorrichtung (30; 31) abhängig von einer bestimmten Sequenz eine Teilmenge der Parameter bereitgestellt wird, und
**dass** die Teilmenge diejenigen Parameter (51-56) der bestimmten Sequenz umfasst, welche von der externen Steuervorrichtung (30; 31) bezüglich der bestimmten Sequenz veränderbar sind.

12. Verfahren nach Anspruch 10 oder 11,
**dadurch gekennzeichnet,**
**dass** die Parameterinformation zumindest eine Information aus einer Informationsgruppe umfasst, welche folgende Informationen umfasst:
• eine Information über die Verfügbarkeit und Änderbarkeit des Parameters,
• eine Bezeichnung des Parameters,
• einen Typ und eine Einheit des Parameters,
• gültige Werte des Parameters,
• eine Information über einen einstellbaren Wertebereich des Parameters,
• eine detaillierte Beschreibung des Parameters, und
• eine eindeutige Identifikation des Parameters.

13. Verfahren nach einem der Ansprüche 10-12,
**dadurch gekennzeichnet,**
**dass** mittels der externen Steuervorrichtung (30; 31) eine Teilmenge der Parameter (51-56) erzeugbar oder änderbar ist, welche diejenigen Parameter einer Sequenz umfasst, die von der externen Steuervorrichtung (30; 31) bezüglich dieser Sequenz veränderbar sind.

14. Verfahren nach Anspruch 2 und einem der Ansprüche 10-13,
**dadurch gekennzeichnet,**
**dass** abhängig von den Autorisierungsdaten jeder der Parameter mittels der externen Steuervorrichtung (30; 31) änderbar ist.

15. Magnetresonanzanlage, welche einen Grundfeldmagneten (1), ein Gradientenfeldsystem (3), mindestens eine HF-Antenne (4), eine Schnittstelle zur Kommunikation mit einer externen Steuervorrichtung und eine Steuervorrichtung (10) zur Ansteuerung des Gradientenfeldsystems (3) und der mindestens einen HF-Antenne (4), zum Empfang von von der mindestens einen HF-Antenne (4) aufgenommenen MR-Signalen (25) und zur Auswertung der MR-Signale umfasst,
wobei die Magnetresonanzanlage ausgestaltet ist, um über die Schnittstelle eine Anweisung der externen Steuervorrichtung zu erfassen und die Anweisung auszuführen,
**dadurch gekennzeichnet,**
**dass** die Magnetresonanzanlage (5) ausgestaltet ist, um über die Schnittstelle eine Kommunikationsverbindung (25) mit der externen Steuervorrichtung (30; 31) zu erstellen, was ein Erfassen von Autorisierungsdaten der externen Steuervorrichtung (30; 31) umfasst, wobei die Autorisierungsdaten eine Lizenz aufweisen, die angibt, welche Dienste (42-47) oder Anweisungen in der Magnetresonanzanlage (5) von der externen Steuervorrichtung (30; 31) ausgeführt werden können, und
**dass** die Magnetresonanzanlage (5) ausgestaltet ist, um die erfasste Anweisung abhängig von einer Berechtigung, welche durch die Lizenz definiert wird, auszuführen.

16. Magnetresonanzanlage nach Anspruch 15,
**dadurch gekennzeichnet,**
**dass** die Magnetresonanzanlage zur Durchführung des Verfahrens nach einem der Schritte 1-14 ausgestaltet ist.

17. System, welches eine Magnetresonanzanlage nach Anspruch 15 oder 16 und eine externe Steuervorrichtung umfasst,
wobei die Steuervorrichtung eine Steuerung, eine Schnittstelle zur Kommunikation mit einer Magnetresonanzanlage und eine Anzeige umfasst,
wobei die Steuervorrichtung ausgestaltet ist, um über die Schnittstelle eine Kommunikationsverbindung mit der Magnetresonanzanlage zu erstellen und um eine Anweisung über die Schnittstelle an die Magnetresonanzanlage zu senden.

18. Computerprogrammprodukt, welches ein Programm umfasst und direkt in einen Speicher einer programmierbaren Steuereinrichtung (10) einer Magnetresonanzanlage (5) ladbar ist, mit Programm-Mitteln, um alle Schritte des Verfahrens nach einem der Ansprüche 1-14 auszuführen, wenn das Programm in der Steuereinrichtung (10) der Magnetresonanzanlage (5) ausgeführt wird.

19. Elektronisch lesbarer Datenträger mit darauf gespeicherten elektronisch lesbaren Steuerinformationen, welche derart ausgestaltet sind, dass sie bei Verwendung des Datenträgers (21) in einer Steuereinrichtung (10) einer Magnetresonanzanlage (5) das Verfahren nach einem der Ansprüche 1-14 durchführen.

## Claims

1. Method for operating a magnetic resonance facility (5) by an external control device (30; 31),
wherein the magnetic resonance facility (5) comprises an interface (24) for communication with the external control device (30; 31), wherein the magnetic resonance facility (5) carries out the following steps:
establishing a communications link (25) between the external control device (30; 31) and the magnetic resonance facility (5) via the interface (24),
acquiring an instruction from the external control device (30; 31) via the interface (24), and
carrying out the instruction on the magnetic resonance facility (5),
**characterised in that**
the establishment of the communications link (25) includes acquisition of authorisation data from the external control device (30; 31), wherein the authorisation data comprises a license that states which services (42-47) or instructions in the magnetic resonance facility (5) can be carried out by the external control device (30; 31), and
the instruction acquired by the magnetic resonance facility (5) is carried out depending on an authorisation that is defined by the license.

2. Method according to claim 1,
**characterised in that**
a plurality of types of authorisation exist,
the authorisation includes at least one of the types of authorisation,
each instruction of the magnetic resonance facility (5) and/or each service (42-47) of the magnetic resonance facility (5) is assigned to one of the types of authorisation, and
the instruction from the magnetic resonance facility (5) that has been acquired is only carried out if the authorisation has the type of authorisation that is assigned to the instruction and/or that is assigned to a service (42-47) that is assigned to the instruction.

3. Method according to one of the preceding claims,
**characterised in that**
the magnetic resonance facility (5) establishes a plurality of communications links (25) to a plurality of external control devices (30, 31), such that in each case a plurality of communications links (25) between an external control device (30; 31) and the magnetic resonance facility (5) are available at the same time.

4. Method according to one of the preceding claims,
**characterised in that**
an information service (45) is provided by the magnetic resonance facility (5) via the interface (24), with which service at least one of the following instructions can be carried out:
acquiring a list of sequences that are available to the magnetic resonance facility (5),
acquiring a current status of a specific sequence, and
acquiring a remaining scanning time for a sequence that is currently running on the magnetic resonance facility (5).

5. Method according to one of the preceding claims,
**characterised in that**
a control service (46) is provided by the magnetic resonance facility (5) via the interface (24), with which at least one of the following instructions can be carried out:
opening a specific sequence,
closing a specific sequence,
starting a sequence that has been opened, and
aborting a sequence that has been started.

6. Method according to one of the preceding claims,
**characterised in that**
a change service (44) is provided by the magnetic resonance facility (5) via the interface (24), with which service at least one of the following instructions can be carried out:
changing at least one parameter in a specific sequence, and
stopping a sequence that is running, and
continuing a sequence that has been stopped.

7. Method according to one of the preceding claims,
**characterised in that**
a patient data service (43) is provided by the magnetic resonance facility (5) via the interface (24), via which at least one of the following information items can be acquired:
the patient's name,
the patient's age,
the patient's body length, and
the patient's weight.

8. Method according to one of the preceding claims,
**characterised in that**
an interactive service (47) is provided by the magnetic resonance facility (5) via the interface (24), which service forwards to the external control device (30; 31) a report that is issued by the magnetic resonance facility (5).

9. Method according to claim 8,
**characterised in that**
the report requires a confirmation so that a sequence that is currently running in the magnetic resonance facility (5) is continued, and
this confirmation is acquired by the external control device (5).

10. Method according to one of the preceding claims,
**characterised in that**
a parameter service is provided by the magnetic resonance facility via the interface, which service provides the external control device (30; 31) with an interface in order to acquire parameter information via said interface that relates to parameters (51-56) of sequences in the magnetic resonance facility (5).

11. Method according to claim 10,
**characterised in that**
depending on a specific sequence, the external control device (30; 31) is provided by the magnetic resonance facility (5) with a subset of the parameters, and that
the subset includes those parameters (51-56) in the specific sequence that may be changed by the external control device (30; 31) with respect to the specific sequence.

12. Method according to claim 10 or 11,
**characterised in that**
the parameter information includes at least one item of information from an information set that includes the following information:
• an item of information on the availability and modifiability of the parameter,
• a designation of the parameter,
• a type and a unit for the parameter,
• valid values for the parameter,
• an item of information on a selectable value range for the parameter.
• a detailed description of the parameter.
• a clear identification of the parameter.

13. Method according to one of claims 10-12,
**characterised in that**
by means of the external control device (30; 31), a subset of the parameters (51-56) can be generated or changed, which subset includes those parameters in a sequence that can be changed by the external control device (30; 31) with respect to this sequence.

14. Method according to claim 2 and one of claims 10-13,
**characterised in that**
depending on the authorisation data, any of the parameters can be changed by means of the external control device (30; 31).

15. Magnetic resonance facility, which includes a basic field magnet (1), a gradient field system (3), at least one HF antenna (4), an interface for communicating with an external control device, and a control device (10) to activate the gradient field system (3) and the at least one HF antenna (4), for receiving MR signals (25) received by the HF antenna/antenna and for evaluating the MR signals,
wherein the magnetic resonance facility is designed to acquire via the interface an instruction from the external control device and to carry out the instruction,
**characterised in that**
the magnetic resonance facility (5) is designed to establish, via the interface, a communications link (25) with the external control device (30; 31), which includes acquisition of authorisation data from the external control device (30; 31), wherein the authorisation data comprises a license that states which services (42-47) or instructions in the magnetic resonance facility (5) can be carried out by the external control device (30; 31), and
the magnetic resonance facility (5) is designed to carry out the instruction acquired depending on an authorisation that is defined by the license.

16. Magnetic resonance facility according to claim 15,
**characterised in that**
the magnetic resonance facility is designed to carry out the method according to one of steps 1-14.

17. System, including a magnetic resonance facility according to claim 15 or 16 and an external control device,
wherein the control device includes a control, an interface for communicating with a magnetic resonance facility, and a display panel,
wherein the control device is designed to establish a communications link with the magnetic resonance facility via the interface and to send an instruction to the magnetic resonance facility via the interface.

18. Computer program product, which includes a program and can be loaded directly into a memory of a programmable control device (10) of a magnetic resonance facility (5), with programming means to carry out all the steps in the method according to one of claims 1-14 when the program is loaded in the control device (10) of the magnetic resonance facility (5) .

19. Electronically-readable data-carrier, on which is stored electronically readable control data, which is designed such that, when the data-carrier (21) is used in a control device (10) of a magnetic resonance facility (5), the method according to one of claims 1-14 can be carried out.

## Revendications

1. Procédé pour faire fonctionner un système (5) à résonance magnétique par un dispositif (30; 31) extérieur de commande,
dans lequel le système (5) à résonance magnétique a une interface (24) de communication avec le dispositif (30; 31) extérieur de commande, le système (5) à résonance magnétique effectuant les stades suivants :
établissement d'une liaison (25) de communication entre le dispositif (30; 31) extérieur de commande et le système (5) à résonance magnétique par l'interface (24),
détection d'une instruction du dispositif (30; 31) extérieur de commande par l'intermédiaire de l'interface (24) et
exécution de l'instruction sur le système (5) à résonance magnétique,
**caractérisé**
**en ce que** l'établissement de la liaison (25) de communication comprend une détection de données d'autorisation du dispositif (30; 31) extérieur de commande, les données d'autorisation ayant une licence, qui indique les services (42 à 47) ou les instructions, qui peuvent être exécutés dans le système (5) à résonance magnétique par le dispositif (30; 31) extérieur de commande et
**en ce que** l'instruction détectée est exécutée par le système (5) à résonance magnétique en fonction d'une autorisation définie par la licence.

2. Procédé suivant la revendication 1,
**caractérisé**
**en ce qu'**il y a plusieurs types d'autorisation,
**en ce que** l'autorisation comprend au moins l'un des types d'autorisation,
**en ce que** chaque instruction du système (5) à résonance magnétique et/ou chaque service (42 à 47) du système (5) à résonance magnétique est associé à l'un des types d'autorisation et
**en ce que** l'instruction détectée est réalisée par le système (5) à résonance magnétique si l'autorisation a le type d'autorisation associé à l'instruction et/ou le type d'autorisation associé à un service (42, 47) associé à l'instruction.

3. Procédé suivant l'une des revendications précédentes, **caractérisé**
**en ce que** le système (5) à résonance magnétique établit plusieurs liaisons (25) de communication vers plusieurs dispositifs (30, 31) extérieurs de commande, de manière à avoir, simultanément, plusieurs liaisons (25) de communication entre, respectivement, un dispositif (30; 31) extérieur de commande et le système (5) à résonance magnétique.

4. Procédé suivant l'une des revendications précédentes,
**caractérisé**
**en ce qu'**il est mis à disposition, par le système (5) de résonance magnétique, par l'intermédiaire de l'interface (24), un service (45) d'information par lequel au moins l'une des instructions suivantes peut être réalisée :
détection d'une liste de séquences, qui sont disponibles pour le système (5) à résonance magnétique,
détection d'un état présent d'une séquence déterminée et
détection d'un temps de mesure restant d'une séquence passant présentement sur le système (5) à résonance magnétique.

5. Procédé suivant l'une des revendications précédentes,
**caractérisé**
**en ce qu'**il peut être mis à disposition, par le système (5) à résonance magnétique, par l'interface (24), un service (46) de commande par lequel au moins l'une des instructions suivantes peut être réalisée :
ouverture d'une séquence déterminée,
fermeture d'une séquence déterminée,
lancement d'une séquence ouverte et
interruption d'une séquence lancée.

6. Procédé suivant l'une des revendications précédentes,
**caractérisé**
**en ce qu'**il peut être mis à disposition, par le système (5) à résonance magnétique, par l'intermédiaire de l'interface (24), un service (44) de modification par lequel au moins l'une des instructions suivantes peut être réalisée :
modification d'au moins un paramètre d'une séquence déterminée et
maintien d'une séquence qui passe et
continuation d'une séquence arrêtée.

7. Procédé suivant l'une des revendications précédentes, **caractérisé**
**en ce qu'**il peut être mis à disposition, par le système (5) à résonance magnétique, par l'intermédiaire de l'interface (24), un service (43) de données de patient par lequel au moins l'une des informations suivant peut être détectée :
le nom du patient,
l'âge du patient,
la taille du patient et
le poids du patient.

8. Procédé suivant l'une des revendications précédentes,
**caractérisé**
**en ce qu'**il peut être mis à disposition, par le système (5) à résonance magnétique, par l'intermédiaire de l'interface (24), un service (47) d'interaction, qui achemine, vers le dispositif (30; 31) extérieur de commande, un message donné par le système (5) à résonance magnétique.

9. Procédé suivant la revendication 8,
**caractérisé**
**en ce que** le message demande une confirmation afin de continuer à réaliser une séquence présente du système (5) à résonance magnétique et
la confirmation est détectée par le dispositif (5) extérieur de commande.

10. Procédé suivant l'une des revendications précédentes,
**caractérisé**
**en ce qu'**il est mis à disposition, par le système à résonance magnétique, par l'intermédiaire de l'interface, un service de paramètres, qui met à disposition du dispositif (30; 31) extérieur de commande une interface afin de détecter, par cette interface, des informations se rapportant à des paramètres (51 à 56) de séquences du système (5) à résonance magnétique.

11. Procédé suivant la revendication 10,
**caractérisé**
**en ce qu'**il est mis à disposition, par le système (5) à résonance magnétique du dispositif (30; 31) extérieur de commande, en fonction d'une séquence déterminée, un sous-ensemble des paramètres et
**en ce que** le sous-ensemble comprend les paramètres (51 à 56) de la séquence déterminée, qui peuvent, par le dispositif (30; 31) extérieur de commande, être modifiés en ce qui concerne la séquence déterminée.

12. Procédé suivant la revendication 10 ou 11,
**caractérisé**
**en ce que** l'information de paramètres comprend au moins une information choisie dans le groupe d'informations, qui comprend les informations suivantes :
• une information sur la disponibilité et la possibilité de modifier le paramètre,
• une caractérisation du paramètre,
• un type et une unité du paramètre,
• des valeurs valables du paramètre,
• une information sur une plage de valeur réglable du paramètre,
• une description détaillée du paramètre et
• une identification univoque du paramètre.

13. Procédé suivant l'une des revendications 10 à 12,
**caractérisé**
**en ce qu'**il peut être produit ou modifié, au moyen du dispositif (30; 31) extérieur de commande, un sous-ensemble des paramètres (51 à 56), qui comprend les paramètres d'une séquence, qui peuvent, par le dispositif (30; 31) extérieur de commande, être modifiés en ce qui concerne cette séquence.

14. Procédé suivant la revendication 2 et l'une des revendications 10 à 13,
**caractérisé**
**en ce qu'**en fonction des données d'autorisation, chacun des paramètres peut être modifié au moyen du dispositif (30; 31) extérieur de commande.

15. Système à résonance magnétique, qui comprend un aimant (1) de champ de base, un système (3) de champ non rotationnel, au moins une antenne (4) HF, une interface de communication avec un dispositif extérieur de commande et un dispositif (10) de commande, pour commander le système (3) de champ non rotationnel et la au moins une antenne HF (4), pour recevoir des signaux MR (25), reçus par la au moins une antenne HF (4) et exploiter les signaux MR,
dans lequel le système à résonance magnétique est conformé pour détecter une instruction du dispositif extérieur de commande par l'interface et pour exécuter l'instruction, **caractérisé**
**en ce que** le système (5) à résonance magnétique est conformé pour établir, par l'intermédiaire de l'interface, une liaison (25) de communication avec le dispositif (30; 31) extérieur de commande, ce qui comprend une détection de données d'autorisation du dispositif (30; 31) extérieur de commande, les données d'autorisation ayant une licence, qui indique les services (42 à 47) ou les instructions, qui peuvent être exécutées dans le système (5) à résonance magnétique par le dispositif (30; 31) extérieur de commande et
**en ce que** le système (5) à résonance magnétique est conformé pour exécuter l'instruction détectée en fonction d'une autorisation définie par la licence.

16. Système à résonance magnétique suivant la revendication 15,
**caractérisé**
**en ce que** le système à résonance magnétique est conformé pour effectuer le procédé suivant l'un des stades 1 à 14.

17. Ensemble, qui comprend un système à résonance magnétique suivant la revendication 15 ou 16 et un dispositif extérieur de commande, dans lequel le dispositif de commande comprend une commande, une interface de communication avec un système à résonance magnétique et un affichage,
dans lequel le dispositif de commande est conformé pour établir, par l'intermédiaire de l'interface, une liaison de communication avec le système à résonance magnétique et pour envoyer une instruction par l'intermédiaire de l'interface au système à résonance magnétique.

18. Produit de programme d'ordinateur, qui comprend un programme et qui peut être chargé directement dans une mémoire d'un dispositif (10) de commande programmable d'un système (5) à résonance magnétique, comprenant des moyens de programme pour effectuer tous les stades du procédé suivant l'une des revendications 1 à 14 lorsque le programme est réalisé dans le dispositif (10) de commande du système (5) à résonance magnétique.

19. Support de données déchiffrable électroniquement, ayant des informations de commande, qui sont mémorisées et qui peuvent être déchiffrées électroniquement, lesquelles sont conformées de manière à ce qu'elles effectuent, lors de l'utilisation du support (21) de données dans un dispositif (10) de commande d'un système (5) à résonance magnétique, le procédé suivant l'une des revendications 1 à 14.
